# EUROPÄISCHE PATENTSCHRIFT

(11) **EP 2 905 598 B1**
(45) Veröffentlichungstag und Bekanntmachung des Hinweises auf die Patenterteilung: **17.06.2020**
(21) Anmeldenummer: 15000333.3
(22) Anmeldetag: 05.02.2015
(51) Int. Cl.: G01N 1/20, G01N 33/18

(54) **Mobiles Handgerät zur Trinkwasserprüfung**
Mobile handheld device for testing drinking water
Appareil manuel mobile destiné au contrôle de l'eau potable

(30) Priorität: 11.02.2014 DE 102014001716
(43) Veröffentlichungstag der Anmeldung: 12.08.2015
(60) Teilanmeldung: 19217073.6
(73) Patentinhaber: Erwin Quarder Systemtechnik GmbH, 32339 Espelkamp (DE)
(72) Erfinder: Kottkamp, Eike, 32312 Lübbecke (DE)
(74) Vertreter: Aulich, Martin

(56) Entgegenhaltungen:
- WO-A1-2005/052573
- WO-A1-2013/158515
- DE-A1-102012 009 076
- US-A1- 2002 130 069
- US-A1- 2006 020 427

## Beschreibung

Die vorliegende Erfindung betrifft ein mobiles Handgerät zur Messung von Parametern von aus einer Wasserentnahmestelle fließendem Wasser, insbesondere Trinkwasser.

Die Trinkwasserverordnung in Deutschland fordert sowohl für den privaten als auch für den gewerblichen Bereich jährlich durchzuführende Prüfungen der Trinkwasserqualität. Solche Prüfungen umfassen insbesondere die Messung diverser Wasserparameter. Es besteht das Bedürfnis, solche Messungen möglichst zuverlässig, schnell und kostengünstig durchführen zu können.

In der WO 2005/052573 A1 ist ein an einen Hydranten angeschlossenes Gerät offenbart, mit dem bestimmte Wasserparameter gemessen werden können. Die Prüfung der Wasserparameter erfolgt automatisiert über mehrere Tage oder Wochen, ohne dass ein Bediener eingreifen muss. Das Gerät verfügt unter anderem über einen Zulauf, durch den Wasser durch das Gerät fließen kann, und einen Ablauf, durch den das Wasser nach Durchfluss durch das Gerät aus diesem abfließen kann. Das Gerät verfügt weiter über eine Messeinrichtung, mit der Wasserdruck des durchfließenden Wassers gemessen werden kann. Über eine hinterlegte Tabelle kann aus dem Wasserdruck die Zeit bestimmt werden, nach der eine bestimmte, als ausreichend beschriebende Menge Wasser durch das Gerät geflossen ist. Der Durchfluss wird dann mittels einer Steuereinheit, die ein entsprechendes Ventil betätigt, nach Ablauf dieser Zeit gestoppt.

In den Druckschriften US 2006/020427 A1, US 2002/130069 A1 und DE 10 2012 009 076 A1 sind weitere Varianten von Messeinrichtungen zur Messung von Wasserparametern offenbart.

Hiervon ausgehend liegt der Erfindung die Aufgabe zugrunde, für die eingangs genannten Messungen ein geeignetes mobiles Handgerät anzugeben.

Diese Aufgabe wird gelöst durch ein mobiles Handgerät mit den Merkmalen des Anspruchs 1.

Die Erfindung schlägt demnach vor, für die Messung von physikalischen und/oder chemischen und/oder biologischen Wasserparametern von insbesondere Trinkwasser ein mobiles Handgerät einzusetzen, das über mehrere Messeinrichtungen verfügt, mit denen unterschiedliche Wasserparameter gemessen werden können. Im Regelfall umfasst dabei die Wasserentnahmestelle, jedenfalls in Privathaushalten, einen sogenannten Wasserhahn, aus dem das Trinkwasser entnommen werden kann. Die Erfindung sieht vor, das mobile Handgerät zeitweise, nämlich mindestens für die Dauer der Messungen, an den Wasserauslass der zu prüfenden Wasserentnahmestelle anzuschließen, insbesondere an den Wasserauslass eines bzw. des Wasserhahns der Wasserentnahmestelle.

Das Handgerät verfügt erfindungsgemäß über einen Zulauf, durch den das Wasser aus dem Wasserauslass der Wassersentnahmestelle in das Handgerät fließen bzw. strömen kann. Weiter verfügt es über einen Ablauf, durch den das Wasser nach dem Durchfluss durch das Handgerät wieder aus diesem herausfließen kann, insbesondere nachdem es entlang der Messeinrichtungen geführt wurde. Das aus dem Ablauf des Handgerätes herausfließende Wasser kann dann beispielsweise in Richtung eines unter dem Handgerät angeordneten Waschbeckens oder dergleichen abfließen.

Weiter weist das Handgerät ein Ventil auf, mit dem der Durchfluss des Wassers durch das Handgerät bzw. das Abfließen des Wassers aus dem Ablauf gesperrt bzw. gestoppt werden kann.

Jedenfalls eine der Messeinrichtungen ist erfindungsgemäß ein Wassermengenzähler. Dieser ist wichtig, um für die spätere Entnahme von Wasserproben mithilfe von separaten Probengefäßen an unterschiedlichen Wasserentnahmestellen definierte, jeweils vergleichbare Ausgangsbedingungen schaffen zu können, wie dies später noch näher erläutert wird.

Der Wassermengenzähler ist dabei so ausgebildet, dass mit ihm die Menge (bzw. das Volumen) des Wassers bestimmt werden kann, die seit dem Beginn der entsprechenden Wassermengenmessung durch das an den Wasserauslass angeschlossene Handgerät bzw. entlang des Wassermengenzählers fließt.

Zweckmäßigerweise wird das Handgerät für die nachfolgende Wassermengenmessung zunächst an den Wasserauslass angeschlossen, dann ggf. das Ventil der Wasserentnahmestelle - regelmäßig ein handelsüblicher Wasserhahn - geöffnet und die Wassermengenmessung anschließend bevorzugt durch einen Bediener des Handgeräts in nachfolgend noch näher beschriebener Weise gestartet. Dies in der Regel zum Zeitpunkt der Öffnung des Wasserhahns oder zu einem beliebigen späteren Zeitpunkt.

Das Handgerät weist eine elektronische Steuereinheit auf, mit der das Absperrventil des Handgeräts betätigt bzw. geöffnet und geschlossen werden kann. Erfindungsgemäß ist nun vorgesehen, dass die elektronische Steuereinheit des Handgeräts das Absperrventil automatisch schließt, sodass der Wasserfluss durch das Handgerät und somit letztlich auch der weitere Wasserfluss aus der Wasserentnahmestelle unterbrochen wird, wenn die Messungen des Wassermengenzählers ergeben, dass die Wassermenge, die seit Beginn der Messungen durch das Handgerät geströmt sind, eine vorbestimmte Bedingung erfüllt. Beispielsweise kann der Durchfluss des Wassers durch das Handgerät auf diese Weise gestoppt werden, wenn eine bestimmte, vordefinierte Wassermenge, beispielsweise gemessen in Litern, durch das Handgerät geströmt ist.

Hierdurch können definierte Voraussetzungen geschaffen werden, unter denen dann der Wasserentnahmestelle im Folgenden beispielsweise eine Wasserprobe entnommen werden kann. Wenn beispielsweise die Wassermenge, ab der das Ventil des Handgeräts automatisch schließt, standardmäßig auf einen Wert von drei Litern eingestellt wird, könnte jede Wasserprobeentnahme jeder Wasserentnahmestelle automatisch unter vergleichbaren Ausgangsbedingungen erfolgen, nämlich dann, wenn aus der Wasserentnahmestelle bereits (mindestens) drei Liter geflossen sind. Hintergrund einer solchen Maßnahme ist, dass regelmäßig für Probeentnahmen nicht das Restwasser genommen werden darf, das sich bei Beginn der Messungen noch in den Leitungen befindet, an die die Wasserentnahmestelle angeschlossen ist, sondern insofern nachlaufendes "frisches" Wasser aus der eigentlichen Wasserquelle, beispielsweise einem Frischwassertank.

Der Start der oben beschriebenen Wassermengenmessung kann beispielsweise durch Betätigung einer von dem Bediener betätigbaren Eingabeeinrichtung des Handgeräts erfolgen, die mit der Steuereinheit wirkverbunden ist bzw. über die die Steuereinheit bedien- bzw. beeinflussbar ist.

So kann vorgesehen sein, dass das Absperrventil des Handgerätes bei erstmaligem Anschluss an die Wasserentnahmestelle zunächst standardmäßig geschlossen ist. Anschließend öffnet der Bediener das Ventil des Wasserauslasses bzw. den Wasserhahn. Zum Start der nachfolgenden Wassermengenmessung betätigt der Bediener die Eingabeeinrichtung, indem er etwa ein entsprechendes Betätigungsorgan der Eingabeeinrichtung betätigt, wie etwa eine bestimmte Taste etc.

Hierdurch getriggert öffnet die Steuereinheit zum einen das Absperrventil, zum anderen sendet sie der Wassermengenmesseinrichtung ein entsprechenden Startsignal, sodass diese ihre Messungen beginnt. Die Steuereinheit überwacht bzw. prüft anschließend, ob mindestens einer der Messwerte, die der Wassermengenzähler liefert, die vorgegebene Bedingung erfüllt. Sobald dies der Fall ist, schließt sie das Absperrventil.

Unmittelbar nach dem Schließen des Absperrventils oder aber nachdem mittels der oder den anderen Messeinrichtungen des Handgeräts zusätzlich eine oder mehrere weitere Messungen durchgeführt wurden, kann dann der Wasserentnahmestelle die Wasserprobe entnommen werden, beispielsweise um diese in einem Labor separat untersuchen zu können, beispielsweise auf Verkeimung, etwa durch Legionellen.

Für die eigentliche Probennahme, d.h. das Abfüllen einer bestimmten Wassermenge in ein entsprechendes Gefäß, wird das Handgerät vorteilhafterweise zunächst von dem Wasserauslass abgenommen bzw. von diesem entfernt.

Das mobile Handgerät verfügt insgesamt, wie oben bereits angedeutet, über mindestens eine, bevorzugt mehrere weitere Messeinrichtungen, die zur Analyse des Wassers sinnvoll bzw. sehr hilfreich sind.

Bevorzugt weist das Handgerät beispielsweise neben dem Wassermengenzähler eine Messeinrichtung auf zur Messung des statischen Leitungsdrucks in der Wasserleitung, an die die Wasserentnahmestelle angeschlossen ist.

Weiter kann das Handgerät einen Durchflussmesser zur Messung der pro Zeiteinheit aus der Wasserentnahmestelle fließenden Wassermenge aufweisen.

Auch kann das Handgerät über eine Temperaturmesseinrichtung verfügen, sodass die Temperatur des aus der Wasserentnahmestelle stammenden Wassers messbar ist.

Weitere Messeinrichtungen des Handgeräts können zur Messung des pH-Werts, zur Messung der elektrischen Leitfähigkeit und/oder zur Messung von (sonstigen) chemischen oder ggf. biologischen Eigenschaften des Wassers dienen bzw. ausgebildet sein.

Gemäß einer wichtigen Weiterbildung der Erfindung ist vorgesehen, dass das Handgerät an verschiedenste Arten von Wasserauslässen von Wasserentnahmestellen, insbesondere von Wasserauslässen von Wasserhähnen derselben, angeschlossen werden kann. Vorgesehen ist zu diesem Zweck die Verwendung eines separaten Adapters, der einerseits mit dem Wasserauslass der Wasserentnahmestelle lösbar verbunden werden kann, andererseits mit dem Zulauf des Handgeräts. Der Adapter ist entsprechend sowohl an den Zulauf des Handgeräts als auch an den Wasserauslass der Wasserentnahmestelle angepasst. Bevorzugt weist der Adapter für den Anschluss desselben an den Wasserauslass einen Schnellverschluss auf, beispielsweise einen Bügelverschluss oder einen Klemmverschluss, sodass der Adapter in einfacher und vor allem schneller Art und Weise mit dem Wasserauslass verbunden werden kann.

Erfindungsgemäß ist für die Messungen des Handgeräts jeweils ein separates, bevorzugt steriles Wasserführungsteil vorgesehen, das in den Zulauf des Handgeräts eingebracht wird, bevor das Handgerät an den Wasserauslass angeschlossen wird. Bevorzugt ist das Wasserführungsteil hierzu in den Zulauf einschraubbar bzw. eingeschraubt oder allgemein kraft- und/oder formschlüssig mit dem Zulauf verbunden. Entsprechend ist der Zulauf des Handgeräts so ausgebildet, dass es ein solches separates Wasserführungsteil aufnehmen kann. Hierdurch kann eine (Rück-)Verkeimung des Wasserauslasses bzw. der Wasserentnahmestelle durch das Handgerät begrenzt oder verhindert werden.

Das Wasserführungsteil wird dabei so platziert, dass nach Verbindung des Handgeräts mit dem Wasserauslass (ggf. über den Adapter) das aus dem Wasserauslass fließende Wasser durch das Wasserführungsteil in das Innere des Handgeräts geführt wird, nämlich zu oder entlang der Messeinrichtungen. Und zwar bevorzugt, ohne dass wasserführende Flächen bzw. Teile des Wasserauslasses direkt mit den übrigen Teilen des Handgeräts in Berührung kommen können.

Bevor mit dem Handgerät nach erfolgten Messungen neue Messungen an einer anderen Wasserentnahmestelle durchgeführt werden, kann dann das Wasserführungsteil aus dem Zulauf entnommen, separat sterilisiert und anschließend wieder eingesetzt werden. Oder es kann schlicht durch ein neues, steriles Wasserführungsteil ersetzt werden. In diesem Fall wäre das Wasserführungsteil jeweils als Einweg-Produkt ausgebildet. Bei einem solchen Wasserführungsteil kann es sich beispielsweise um ein rohrartiges bzw. röhrchenartiges Teil handeln.

Hintergrund dieser Maßnahme ist, dass das Handgerät, insbesondere dessen Messeinrichtungen im Inneren, nicht oder nur sehr schwierig sterilisiert werden können.

In weiterer Ausbildung der Erfindung verfügt das Handgerät im Übrigen über eine Anzeigeeinheit, über die dem Bediener Ergebnisse von durchgeführten Messungen anzeigbar sind und/oder über eine Sendeeinheit, mit der die Ergebnisse der durchgeführten Messungen, insbesondere drahtlos, an eine entfernte Empfangseinheit gesendet werden können. Natürlich ist auch denkbar, die Ergebnisse in einem internen Speicher des Handgeräts abzuspeichern. Im Hinblick auf die Verarbeitung, Auswertung und Weiterleitung der Messergebnisse gibt es naturgemäß vielfältige Möglichkeiten.

Was weitere automatische oder teilautomatische Steuerungs- bzw. Programmabläufe des Handgeräts betrifft, gibt es naturgemäß verschiedenste Möglichkeiten.

Bevorzugt ist unter anderem vorgesehen, dass nach dem oben erwähnten automatischen Schließvorgang des Absperrventils, wenn also die durch das Handgerät durchgeflossene Wassermenge die vorbestimmte Bedingung erfüllt hat, mittels der Messeinrichtung des Handgeräts zur Messung des statischen Wasserdrucks automatisch der statische Wasserdruck gemessen wird, der stromauf des Ventils und somit in der Wasserleitung anliegt, an die die Wasserentnahmestelle angeschlossen ist. Mit anderen Worten wird durch die Steuereinheit nach dem Schließen des Ventils automatisch die vorgenannte Messeinrichtung getriggert, sodass diese beginnt die Messungen des statischen Wasserdrucks vorzunehmen.

Ein besonderes Ablaufprogamm umfasst folgende Maßnahmen:
Beispielsweise kann im ersten Schritt die oben erwähnte Wassermengenmessung in einem Kaltwasserfluss erfolgen, indem das Ventil der Wasserentnahmestelle bzw. der Wasserhahn von einem Bediener so eingestellt wird, dass aus dem Wasserauslass entsprechend zunächst kaltes Wasser strömt, bis die Steuereinheit das Absperrventil aufgrund der von der Wasserdurchflussmenge erfüllten Bedingung in der oben beschriebenen Weise schließt.

Bevorzugt nach diesem Schließvorgang des Absperrventils, insbesondere nachdem, wie oben beschrieben, die (Kalt-)Wasserprobe entnommen wurde und ggf. automatisch auch der statische Kaltwasserleitungsdruck und ggf. auch die Kaltwassertemperatur und ggf. zudem weitere Wasserparameter des Kaltwassers gemessen wurden, stellt der Bediener den Wasserhahn auf die Stellung "Warmwasser". Insofern das Handgerät zuvor von dem Wasserauslass abgenommen wurde, schließt er es wieder an, insbesondere mithilfe des Adapters mit Schnellverschluss.

Der Bediener betätigt dann die Eingabeeinrichtung, wodurch das Absperrventil, getriggert durch die mit der Eingabeeinrichtung zusammenwirkende Steuereinheit, wieder geöffnet wird, sodass das Warmwasser durch das Handgerät hindurch läuft und schließlich aus dem Ablauf des Handgeräts herausläuft.

Getriggert durch die Steuereinheit wird dann mittels der Temperaturmesseinrichtung des Handgeräts automatisch mehrfach die Temperatur des aus der Wasserentnahmestelle fließenden Warmwassers gemessen, insbesondere kontinuierlich.

Das Absperrventil wird dann, ebenfalls automatisch getriggert durch die Steuereinheit, (erneut) geschlossen, wenn die Temperaturmessungen ergeben, dass die Wassertemperatur des Warmwassers einer vorbestimmten Bedingung entspricht, insbesondere in einer vorbestimmten Weise stabil ist. Als stabil kann sie von der Steuereinheit beispielsweise dann bewertet werden, wenn sich die gemessenen Temperaturwerte über einen definierten Messzeitraum nicht mehr oder nur innerhalb eines Toleranzbereichs ändern.

Weiter kann vorgesehen sein, dass nach dem vorgenannten erneuten Schließvorgang des Ventils der statische Wasserdruck, diesmal derjenige des Warmwassers, mittels der Messeinrichtung des Handgeräts zur Messung des statischen Wasserdrucks erneut automatisch, d.h. getriggert durch die Steuereinheit, gemessen wird. Also derjenige statische Druck, der stromauf des Absperrventils und somit diesmal entsprechend in der Warmwasserleitung anliegt, an die die Wasserentnahmestelle angeschlossen ist. Auch kann vorgesehen sein, das automatisch ggf. zudem in der oben beschriebenen Weise weitere Wasserparameter des Warmwassers gemessen werden.

Nach Abschluss der jeweiligen oder nach Abschluss mehrerer oder sämtlicher Messungen kann/können auf der Anzeigeeinheit des Handgeräts automatisch ein oder mehrere Ergebnisse der durchgeführten Messungen angezeigt werden. Alternativ oder zusätzlich kann vorgesehen sein, dass automatisch ein oder mehrere Messergebnisse der durchgeführten Messungen an eine entfernte Empfangseinheit gesendet werden.

Im Übrigen kann vorgesehen sein, dass der Bediener durch den Mess- bzw. im Programmablauf des Handgeräts interaktiv mittels entsprechender, auf der Anzeigeeinheit des Handgeräts erscheinender Anweisungen geführt wird. Beispielsweise, indem er jeweils dazu aufgefordert wird, ein oder mehrere bestimmte Betätigungsorgane der Eingabeeinrichtung zu betätigen, um etwa das Handgerät bzw. die Steuereinheit dazu zu bringen, dass dieses die Wassermengenmessung im Kaltwasserfluss startet oder etwa die Temperaturmessungen des Warmwassers.

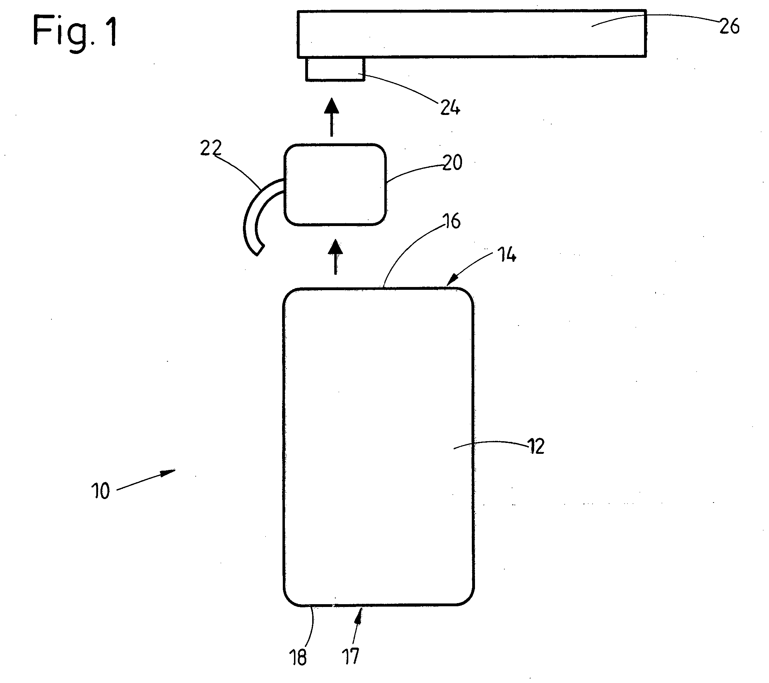

In den beigefügten Prinzipskizzen der Fig. 1-2, ist nur beispielhaft ein Handgerät 10 gezeigt, das in der vorstehend beschriebenen Weise arbeiten kann.

Das dargestellte Handgerät 10 verfügt über ein von einem Bediener gut in einer Hand zu haltendes Gehäuse 12 mit einem nicht dargestellten Zulauf im oberen Gehäusebereich 14, der insbesondere an der Gehäuseoberseite 16 angeordnet ist, sowie einem ebenfalls nicht zu erkennenden Ablauf im Bodenbereich 17, der insbesondere an dem Gehäuseboden 18 angeordnet ist.

Der nicht dargestellte Zulauf des Handgeräts 10 kann mit einem separaten, ebenfalls einen geeigneten Zulauf und Ablauf aufweisenden Adapter 20 verbunden werden.

Bevorzugt verfügt der Adapter 20 über mindestens einen mit einem Betätigungsorgan 22 betätigbaren Schnellverschluss. Im vorliegenden Ausführungsbeispiel verfügt er über zwei Schnellverschlüsse.

Der Adapter 20, nämlich dessen Zulauf, kann mittels des ersten Schnellverschlusses beispielsweise mit dem Wasserauslass 24 eines Wasserhahns 26 einer Wasserentnahmestelle, beispielsweise einer Trinkwasserentnahmestelle in einem Privathaus, lösbar verbunden werden.

Mittels eines zweiten Schnellverschlusses kann der Ablauf des Adapters 20 mit dem Zulauf des Handgeräts 10 verbunden werden.

Die beiden Schnellverschlüsse können jeweils beispielsweise eine Klemmverbindung zwischen Wasserauslass 24 und Adapterzulauf bzw. Adapterablauf und Handgerätzulauf herstellen.

Die Messeinrichtungen, die das Handgerät 10 aufweist, um die eingangs beschriebenen Messungen durchführen zu können, beispielsweise der Wassermengenzähler und ggf. die Temperaturmesseinrichtung, aber auch sämtliche anderen Messeinrichtungen, sind nicht gezeigt. Sie sind im Stand der Technik sämtlich bekannt und werden daher auch nicht separat beschrieben. Erfindungsgemäß werden diese Messeinrichtungen in das Handgerät 10 in geeigneter Weise integriert.

Im Inneren weist das Handgerät 10 naturgemäß ein oder mehrere Kanäle auf, entlang dem/der das zu analysierende, aus dem Wasserhahn 26 fließende Wasser der Wasserentnahmestelle geführt wird. Die Messeinrichtungen des Handgeräts 10 sind entsprechend so im Bereich bzw. in der Nähe des/der Kanäle positioniert, dass sie die entsprechenden Messungen ausführen können.

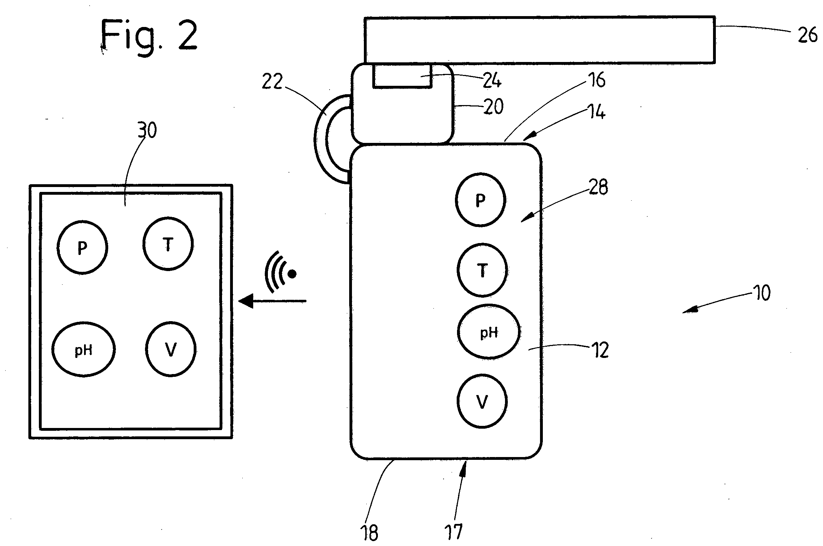

In Fig. 2 ist beispielhaft eine Anzeige- und Eingabeeinrichtung 28 gezeigt, auf der dem Bediener die Messergebnisse angezeigt werden. Die Messergebnisse können beispielsweise mit einer Sendeeinheit des Handgeräts 10 zum Senden von elektromagnetischen Wellen, etwa einem Funkmodul, drahtlos an eine entfernte, entsprechende Empfangseinheit 30 zum Empfangen elektromagnetischer Wellen gesendet werden. Die Anzeige- und Eingabeeinrichtung 28 ist als Touchscreen ausgebildet, sodass hierüber von einem Bediener auch entsprechende Eingaben getätigt werden können, die beispielsweise die oben beschriebenen Steuerungsabläufe triggern.

### Bezugszeichenliste:

- 10: Handgerät
- 12: Gehäuse
- 14: Gehäusebereich
- 16: Gehäuseoberseite
- 17: Bodenbereich
- 18: Gehäuseboden
- 20: Adapter
- 22: Betätigungsorgan
- 24: Wasserauslass
- 26: Wasserhahn
- 28: Eingabeeinrichtung
- 30: Empfangseinheit

## Patentansprüche

1. Mobiles Handgerät (10) zur Messung von Wasserparametern von aus einer Wasserentnahmestelle fließendem Wasser, das an einen Wasserauslass (24) der Wasserentnahmestelle anschließbar ist, mit einem Zulauf, durch den das Wasser der Wasserentnahmestelle nach Anschluss an den Wasserauslass (24) in das mobile Handgerät (10) fließen kann, mit einem Ablauf, durch den das Wasser nach Durchfluss durch das mobile Handgerät (10) aus dem mobilen Handgerät (10) abfließen kann, mit mehreren Messeinrichtungen zur Messung der Wasserparameter, wobei es sich bei einer der Messeinrichtungen um einen Wassermengenzähler handelt, mit dem die durch das mobile Handgerät (10) seit Beginn einer entsprechenden Messung hindurchgeflossene Wassermenge messbar ist, mit einem Ventil, mit dem das Abfließen des Wassers durch den Ablauf des mobilen Handgeräts (10) und damit auch der Durchfluss durch das mobile Handgerät (10) gesperrt werden kann, mit einer elektronischen Steuereinheit, mit der das Ventil betätigbar ist, wobei die Steuereinheit derart ausgebildet ist, dass sie das Ventil automatisch schließt, sodass kein weiteres Wasser aus dem Ablauf des mobilen Handgeräts (10) abfließen kann, wenn Messungen des Wassermengenzählers ergeben, dass eine Wassermenge, die seit Beginn der Messungen durch das Handgerät geflossen ist, einer vorbestimmten Bedingung entspricht, insbesondere einen vorbestimmten Grenzwert erreicht hat, wobei das mobile Handgerät (10) ein bevorzugt steriles Wasserführungsteil, insbesondere ein Einweg-Wasserführungsteil, aufweist, durch das Wasser von der Wasserentnahmestelle in das mobile Handgerät (10) fließen kann, wobei das Wasserführungsteil in den Zulauf des mobilen Handgeräts (10) lösbar eingebracht ist, insbesondere eingesteckt oder eingeschraubt.

2. Mobiles Handgerät gemäß Anspruch 1, **dadurch gekennzeichnet, dass** eine der Messeinrichtungen des mobilen Handgeräts (10) eine Messeinrichtung ist zur Messung eines statischen Leitungsdrucks in einer Wasserleitung, an die die Wasserentnahmestelle angeschlossen ist.

3. Mobiles Handgerät gemäß einem oder mehreren der vorhergehenden Ansprüche, **dadurch gekennzeichnet, dass** eine der Messeinrichtungen des mobilen Handgeräts (10) ein Durchflussmesser ist zur Messung der pro Zeiteinheit durch das mobile Handgerät (10) fließenden Wassermenge.

4. Mobiles Handgerät gemäß einem oder mehreren der vorhergehenden Ansprüche, **dadurch gekennzeichnet, dass** eine der Messeinrichtungen des mobilen Handgeräts (10) eine Messeinrichtung zur Messung der Temperatur des aus der Wasserentnahmestelle fließenden Wassers ist.

5. Mobiles Handgerät gemäß einem oder mehreren der vorhergehenden Ansprüche, **dadurch gekennzeichnet, dass** eine der Messeinrichtungen des mobilen Handgeräts (10) eine Messeinrichtung zur Messung des pH-Wertes des aus der Wasserentnahmestelle fließenden Wassers ist, und/oder dass eine der Messeinrichtungen des mobilen Handgeräts (10) eine Messeinrichtung zur Messung der elektrischen Leitfähigkeit aus der Wasserentnahmestelle fließenden Wassers ist, und/oder dass eine der Messeinrichtungen des mobilen Handgeräts (10) eine Messeinrichtung zur Messung von chemischen Eigenschaften des Wassers ist.

6. Mobiles Handgerät gemäß einem oder mehreren der vorhergehenden Ansprüche, **dadurch gekennzeichnet, dass** das mobile Handgerät (10) über einen oder mehrere Kanäle verfügt, entlang dem/der das Wasser in dem mobilen Handgerät (10) zu den Messeinrichtungen führbar ist.

7. Mobiles Handgerät gemäß einem oder mehreren der vorhergehenden Ansprüche, **dadurch gekennzeichnet, dass** das mobile Handgerät (10) über eine Eingabeeinrichtung (28) verfügt, mit der ein Bediener die elektronische Steuereinheit des mobilen Handgeräts (10) beeinflussende Eingaben tätigen kann.

8. Mobiles Handgerät gemäß einem oder mehreren der vorhergehenden Ansprüche, **dadurch gekennzeichnet, dass** das mobile Handgerät (10) über eine Anzeigeeinheit (28) verfügt, über die einem Bediener Ergebnisse von durchgeführten Messungen anzeigbar sind und/oder eine Sendeeinheit, über die die Ergebnisse der durchgeführten Messungen an eine entfernte Empfangseinheit (30) sendbar sind.

## Claims

1. Mobile handheld device (10) for measuring water parameters of water flowing from a water intake point, which mobile handheld device can be connected to a water outlet (24) of the water intake point, having an inflow, through which the water from the water intake point can flow into the mobile handheld device (10) after connection to the water outlet (24), having an outflow, through which the water can flow out of the mobile handheld device (10) after flowing through the mobile handheld device (10), having a plurality of measuring devices for measuring the water parameters, wherein one of the measuring devices is a water meter which can be used to measure the amount of water which has flowed through the mobile handheld device (10) since the beginning of a corresponding measurement, having a valve which can be used to block the flow of water through the outflow of the mobile handheld device (10) and therefore also the flow through the mobile handheld device (10), having an electronic control unit which can be used to actuate the valve, wherein the control unit is designed in such a manner that it automatically closes the valve, with the result that no further water can flow out of the outflow of the mobile handheld device (10) if measurements by the water meter reveal that an amount of water which has flowed through the handheld device since the beginning of the measurements corresponds to a predetermined condition, in particular has reached a predetermined limit value, wherein the mobile handheld device (10) has a preferably sterile water guiding part, in particular a one-way water guiding part, through which water can flow from the water intake point into the mobile handheld device (10), wherein the water guiding part is releasably introduced, in particular plugged or screwed, into the inflow of the mobile handheld device (10).

2. Mobile handheld device according to Claim 1, **characterized in that** one of the measuring devices of the mobile handheld device (10) is a measuring device for measuring a static pipe pressure in a water pipe, to which the water intake point is connected.

3. Mobile handheld device according to one or more of the preceding claims, **characterized in that** one of the measuring devices of the mobile handheld device (10) is a flowmeter for measuring the amount of water flowing through the mobile handheld device (10) per unit time.

4. Mobile handheld device according to one or more of the preceding claims, **characterized in that** one of the measuring devices of the mobile handheld device (10) is a measuring device for measuring the temperature of the water flowing from the water intake point.

5. Mobile handheld device according to one or more of the preceding claims, **characterized in that** one of the measuring devices of the mobile handheld device (10) is a measuring device for measuring the pH value of the water flowing out of the water intake point, and/or **in that** one of the measuring devices of the mobile handheld device (10) is a measuring device for measuring the electrical conductivity of water flowing out of the water intake point, and/or **in that** one of the measuring devices of the mobile handheld device (10) is a measuring device for measuring chemical properties of the water.

6. Mobile handheld device according to one or more of the preceding claims, **characterized in that** the mobile handheld device (10) has one or more channels, along which the water in the mobile handheld device (10) can be guided to the measuring devices.

7. Mobile handheld device according to one or more of the preceding claims, **characterized in that** the mobile handheld device (10) has an input device (28) which can be used by an operator to make inputs influencing the electronic control unit of the mobile handheld device (10) .

8. Mobile handheld device according to one or more of the preceding claims, **characterized in that** the mobile handheld device (10) has a display unit (28) which can be used to display results of measurements which have been carried out to an operator, and/or a transmitting unit which can be used to transmit the results of the measurements which have been carried out to a remote receiving unit (30).

## Revendications

1. Appareil portatif mobile (10) permettant de mesurer des paramètres d'eau d'une eau coulant depuis un point de prélèvement d'eau, pouvant être branché sur une évacuation d'eau (24) du point de prélèvement d'eau, comprenant une arrivée par laquelle l'eau du point de prélèvement d'eau, après branchement sur l'évacuation d'eau (24), peut couler dans l'appareil portatif mobile (10), un orifice de sortie par lequel l'eau, après passage par l'appareil portatif mobile (10), peut s'écouler depuis l'appareil portatif mobile (10), plusieurs dispositifs de mesure pour mesurer les paramètres d'eau, l'un des dispositifs de mesure étant un compteur de volume d'eau permettant de mesurer le volume d'eau qui est passé par l'appareil portatif mobile (10) depuis le début d'une mesure correspondante, une vanne permettant de bloquer l'écoulement de l'eau à travers l'orifice de sortie de l'appareil portatif mobile (10) et donc également le débit à travers l'appareil portatif mobile (10), une unité de commande électronique permettant d'actionner la soupape, l'unité de commande étant réalisée de telle sorte qu'elle ferme la vanne automatiquement de sorte qu'après écoulement depuis l'appareil portatif mobile (10), plus aucune eau supplémentaire ne peut s'écouler si des mesures du compteur de volume d'eau indiquent qu'un volume d'eau étant passé par l'appareil portatif depuis le début des mesures correspond à une condition prédéterminée, en particulier qu'il a atteint une valeur limite prédéterminée, dans lequel l'appareil portatif mobile (10) présente de préférence une pièce de guidage d'eau stérile, en particulier une pièce de guidage d'eau à usage unique, par laquelle l'eau peut couler depuis le point de prélèvement d'eau dans l'appareil portatif mobile (10), la pièce de guidage d'eau étant insérée de manière amovible dans l'arrivée de l'appareil portatif mobile (10), en particulier enfichée ou vissée.

2. Appareil portatif mobile selon la revendication 1, **caractérisé en ce que** l'un des dispositifs de mesure de l'appareil portatif mobile (10) est un dispositif de mesure pour mesurer une pression de conduite statique dans une conduite d'eau sur laquelle est branché le point de prélèvement d'eau.

3. Appareil portatif mobile selon une ou plusieurs des revendications précédentes, **caractérisé en ce que** l'un des dispositifs de mesure de l'appareil portatif mobile (10) est un débitmètre pour mesurer le volume d'eau coulant à travers l'appareil portatif mobile (10) par unité de temps.

4. Appareil portatif mobile selon une ou plusieurs des revendications précédentes, **caractérisé en ce que** l'un des dispositifs de mesure de l'appareil portatif mobile (10) est un dispositif de mesure pour mesurer la température de l'eau coulant depuis le point de prélèvement d'eau.

5. Appareil portatif mobile selon une ou plusieurs des revendications précédentes, **caractérisé en ce que** l'un des dispositifs de mesure de l'appareil portatif mobile (10) est un dispositif de mesure pour mesurer le pH de l'eau coulant depuis le point de prélèvement d'eau, et/ou **en ce que** l'un des dispositifs de mesure de l'appareil portatif mobile (10) est un dispositif de mesure pour mesurer la conductibilité électrique de l'eau coulant depuis le point de prélèvement d'eau, et/ou **en ce que** l'un des dispositifs de mesure de l'appareil portatif mobile (10) est un dispositif de mesure pour mesurer des propriétés chimiques de l'eau.

6. Appareil portatif mobile selon une ou plusieurs des revendications précédentes, **caractérisé en ce que** l'appareil portatif mobile (10) dispose d'un ou de plusieurs canaux le long desquels l'eau dans l'appareil portatif mobile (10) peut être amenée aux dispositifs de mesure.

7. Appareil portatif mobile selon une ou plusieurs des revendications précédentes, **caractérisé en ce que** l'appareil portatif mobile (10) dispose d'un dispositif d'entrée (28) par lequel un utilisateur peut effectuer des entrées influençant l'unité de commande électronique de l'appareil portatif mobile (10).

8. Appareil portatif mobile selon une ou plusieurs des revendications précédentes, **caractérisé en ce que** l'appareil portatif mobile (10) dispose d'une unité d'affichage (28) permettant d'afficher pour un utilisateur des résultats de mesures effectuées et/ou d'une unité de transmission permettant de transmettre les résultats des mesures effectuées à une unité de réception à distance (30).
